# EUROPEAN PATENT APPLICATION

(11) **EP 2 236 512 A1**
(43) Date of publication of application: **06.10.2010**
(21) Application number: 10170070.6
(22) Date of filing: 18.04.2007
(51) Int. Cl.: C07K 1/22, G01N 33/50, C07K 14/47

(54) **Binding of pathological forms of proteins using conjugated polyelectrolytes**

(62) Divisional of application: 07748414.5
(71) Applicant: Biochromix AB, 171 65 Solna (SE)
(72) Inventor: Åsberg, Peter, SE-113 30, Stockholm (SE); Inganäs, Olle, SE-582 46, Linköping (SE); Anghus, Fredrik, SE-582 33, Linköping (SE)
(74) Representative: Rystedt, Per Hampus

(57) **Abstract**

The invention relates to a method for separation of an aggregated misfolded protein from an environment comprising a non-aggregating normal form of the protein, comprising contacting both said misfolded and normal protein with a conjugated polyelectrolyte (CPE) and separating the CPE/protein complex from the other constituents of the sample.

## Description

### Field of the invention

The present invention relates to the use of conjugated polyelectrolytes for specific, optionally under selective conditions, separation, for example capture, of misfolded, pathological or rogue forms of proteins, i.e. amyloid form, misfolded form or aggregated form, and capture of proteins which are similarly causing aggregation of abnormal forms of proteins which in their normal form are not aggregated. The present invention also relates to a one step method of separating and at the same time detecting the misfolded, pathological or rogue forms of proteins. A further embodiment of the present invention relates to the use of conjugated polyelectrolytes as novel therapeutic agents by interfering with the formation of, or by capturing misfolded, pathological or rogue forms of proteins in vivo.

### Background of the invention

The development of materials and molecules that are capable of selectively capturing misfolded or aggregated forms of proteins, especially the amyloid forms and pathological forms, have received a great deal of attention, owing to their potential for being used as analytic tools in clinical chemistry, in diagnosis, as well as therapeutic agents. Amyloid fibrils are normally stained with small molecule dyes, such as Congo red and thioflavin T. The great drawback of such molecular dyes is that it is not possible to separate, i.e. bind, capture or isolate, the misfolded protein and at the same time detect it.

One methodology for assaying for the presence of amyloid, misfolded or pathological forms of proteins is to subject a sample to proteolysis, for example with proteinase K, for a period sufficient to destroy the native proteins and then perform an immunoassay using an antibody which is not selective for amyloid, misfolded or pathological forms of proteins in the presence of native proteins to determine the presence of said misfolded forms of proteins. If a protease is used to remove native proteins in a sample containing amyloid, misfolded or pathological forms of proteins, this prevents the use of an antibody as a capture agent or as a detection agent during the proteolysis step, since the antibody would naturally be destroyed in the presence of the protease. Thus the protease must be removed or deactivated before the antibody can be introduced. It would be a great advantage to circumvent this limitation on the procedure when capturing and detecting amyloid, misfolded or pathological forms of proteins in the presence of native proteins.

Natural biopolymers, such as proteins, frequently have ordered conformations, such as alpha-helix and beta-sheets, which contribute to the three-dimensional ordered structure and the specific function of the biopolymer. The structure of a protein is essential for the protein's function; it has been shown by many scientists that an unfolded protein may not be functional. More important, in the last few years there is increasing awareness of the danger of protein misfolding and misassembly into for example amyloid and other pathological forms. Misfolding can change a protein from something that is useful into nonfunctional, harmful or even toxic. Human health relies on properly folded protein, and *in vivo* deposition of amyloid fibrils is associated with many diseases of protein conformation, including Alzheimer's disease, Huntington's disease, systemic amyloidoses, and the prion diseases. The prion diseases, i.e. transmissible spongiform encephalopathy (TSE), in animals [e.g. bovine spongiform encephalopathy (BSE), Scrapie and chronic wasting disease (CWD)] and in humans [Creutzfeldt Jakob disease (CJD), Gerstmann-Sträussler-Scheinker disease (GSS), Kuru] are associated with the conformational conversion of the normal cellular prion protein, (PrP^{C}), to an infectious pathogenic disease-associated isoform denoted PrP^{Sc}. Proteins frequently alter their conformation due to different external stimuli and the importance of conformational changes of proteins leading to pathogenic states has been well documented. Especially under conditions that destabilize the native state, proteins can aggregate into characteristic fibrillar assemblies, known as amyloid fibrils. These beta-sheet rich protein assemblies have distinctively different conformations to that of the native state. The misfolded prion protein is even self-propagating (infectious), a property which is entirely encoded within the misfolded conformation. The underlying mechanism of protein misfolding and subsequent amyloid formation is poorly understood. Many lines of evidence support the existence of smaller oligomeric species as intermediates on the pathway from misfolded protein to amyloid. These oligomers vary in morphology, and only a subset of these may be responsible for the cellular toxicity associated with amyloid disease. Furthermore, a single soluble protein can give rise to different "strains" of misfolded product, as evidenced by the yeast prion, Sup35. It has been shown that the infectivity of different yeast prion strains is dependent on the conformation of the infectious protein, and that a single protein can adopt multiple, self-propagating (infectious) conformations. These conformational differences underlie heritable differences in prion strains. In addition, prion strains might also have a major role in determining the specificity of prion transmission.

Chronic human diseases seriously affect the healthcare system. It is well recognized that rapid and accurate diagnostic tools are necessary to afford early intervention and therapy. Only symptomatic therapy is available, like in Alzheimer's disease for example, and these have limited therapeutic efficacy. Presently there are no antemortem molecular diagnostic tests of Alzheimer's disease or transmissible spongiform encephalopathies (TSEs), and the clinical diagnostics that are performed require that disease progression is severe. Further, there are no efficient treatments available yet, and immunotherapy in for example Alzheimer's disease holds great promise. The lack of reliable methods to capture misfolded proteins, monitoring both treatment and disease progression is however a severe shortcoming in treatment of most protein misfolding related diseases.

Hence, there is a need for simple, sensitive and versatile tools that can separate, for example capture, amyloidogenic proteins, such as misfolded prion proteins and different strains of prion proteins, from a sample. There is also a need for novel therapeutic agents by interfering, binding or capturing misfolded or amyloid proteins in vivo.

The possibility to use conjugated polymers (CPs) for studying biological molecules, biomolecular interactions, biomolecular folding events and biomolecular dynamics requires that polymers are compatible with an aqueous environment. This has been accomplished by making conjugated polyelectrolytes (CPEs). As described in many scientific publications CPEs have been utilized for the detection of biomolecules, biomolecular interactions and biomolecular folding events by various means. Examples where CPEs are used to detect biospecific interactions, such as receptor/analyte interactions, through the conformational alterations of the polyelectrolyte chains can be found in the scientific press [Nilsson, K. P. R.; Inganäs, O. Nature Materials 2003, 2, 419-424.; Ho, H-A. et. al. Angew. Chem. Int. Ed. 2002, 41, 1548.; Ho, H-A.; Leclerc, M. J. Am. Chem. Soc. 2004, 126, 1384.; Dore, K.; Dubus, S.; Ho, H-A.; Levesque, I.; Brunette, M.; Corbeil, G.; Boissinot, M.; Boivin, G.; Bergeron, M. G.; Boudreau, D.; Leclerc, M. J. Am. Chem. Soc. 2004, 126, 4240.; WO02/081735, WO03/096016].

### Summary of the invention

Simple methods with improved affinity for capturing misfolded or aggregated forms of proteins, especially the amyloid forms and pathological forms, are required, especially for separating misfolded proteins from samples (ex vivo or in vivo). Methods based on conjugated polyelectrolytes that can capture agents for self-assembled/aggregated forms of proteins, especially misfolded or aggregated forms of proteins, and at the same time acting as transducer reporting the capture event in optical signals, is therefore described in the present invention.

Moreover, the present invention also relates to the use of conjugated polyelectrolytes as novel therapeutic agents which act by interfering, binding or capturing misfolded, amyloid aggregated or pathological protein forms, such as Amyloid beta in Alzheimer's or misfolded prion proteins in TSEs. Conjugated polyelectrolytes bind and capture these protein forms and therefore CPEs influence pathogenesis in living organisms by acting as pharmacophores, i.e. they are therapeutic agents. Conjugated polyelectrolytes that cross the blood brain barrier have an effect on diseases that affect the brain, this include, but is not limited to, Abeta amyloid pathology in living organisms, i.e. influence Alzheimer's disease pathogenesis by acting as therapeutic pharmacophores. The present invention relates to the use of conjugated polyelectrolytes (CPEs), either localized on a solid support or free in solution, for the specific separation, for example capture, of misfolded, pathological or rogue forms of proteins, i.e. amyloid form, misfolded form or aggregated form, and proteins which are similarly aggregating abnormal forms of proteins which in their normal form are not aggregated. Said capture of misfolded proteins using a CPE or several CPEs can optionally be performed under selective conditions for the misfolded protein form. The present invention also relates to a one step method of capturing and detecting, either performed selective under non-selective conditions for misfolded proteins, of the misfolded, pathological or rogue forms of proteins. A further aspect of the present invention is CPEs as tools for in-vivo imaging of misfolded or aggregated forms of proteins, especially the amyloid forms and pathological forms. The invention also relates to in vivo imaging methods, by for example MRI, of misfolded proteins using functionalized conjugated polyelectrolytes.

The present invention solves the shortcomings of the prior art by using CPEs for separation, for example capturing, binding or interfering, of the misfolded proteins in a sample (ex vivo or in vivo), capture and detection of the same, novel optical methods for diagnosis and potential therapeutic agents, all based on conjugated polyelectrolytes. Prior art have demonstrated that CPEs reliably detect amyloid fibrillation in vitro and in ex vivo tissue samples [W02005109005]. The physico chemical reason for capture of amyloidogenic structures using CPEs rely on the repetitive structure of the polyelectrolytes that associated with symmetric repetitive molecular targets (amyloidotic molecules), providing for extremely high affinity. Thus, the selective CPE based methods provides for capture of pathogenic and amyloid proteins associated with a number of diseases. Tools and methods for capture of misfolded or aggregated forms of proteins, especially the amyloid forms and pathological forms, but also therapies of diseases related with these proteins forms are all based on conjugated polyelectrolytes, according to the present invention.

Samples that contain misfolded proteins include, but are not limited to, blood, brain, peripheral tissue, homogenized brain, homogenized peripheral tissue, fluids, biopsies, urine, cerebrospinal fluid (CSF), lymph, plasma, preparations in buffers and biotechnological growth media for protein pharmaceuticals.

One aspect of the invention is the use of a conjugated polyelectrolyte as a capture agent of misassembled/aggregated forms of proteins, especially of amyloid fibrils and pathogenic forms of proteins. The conjugated polyelectrolyte can either be localized on a solid support, on a bead of any size, in a tube, in dialysis equipment, in pumps or be provided free in solution when used as an agent to capture misfolded proteins. The CPE can also have the form of a hydrogel. If the CPE is provided free in solution any means to separate the misfolded protein after being captured by the CPE can be applied, for example separation by sedimentation, centrifugation, adsorption, absorption, drying, boiling or evaporation. Optionally the CPE can be functionalized to provide for means of detection, immobilization, facilitate capture, enhanced selectivity, enhanced affinity or other features of significance for the assay.

Interestingly, conjugated polyelectrolytes, such as poly(thiophene), Poly(3,4-ethylenedioxythiophene) or poly(pyrrole), can be used to detect misfolded proteins [W02005109005]. Sensors based on conjugated polyelectrolytes are sensitive to very minor perturbations, due to amplification by a collective system response and therefore offer a key advantage compared to molecular based sensors of the prior art and the polyelectrolytes also offers a direct detection of the pathogenic prion protein. Combine this direct detection functionality of CPEs with the unique ability to capture misfolded proteins, according to the present invention, and a huge advantage compared to traditional immunohistological techniques is provided as these methods often require the use of a primary antibody for capture and secondary antibody for visualization of the misfolded protein. The possibility to use conjugated polyelectrolytes as capture agents for misfolded proteins requires that polymers are compatible with an aqueous environment and this has been accomplished by making conjugated luminescent polyelectrolytes

A novel way to capture misfolded proteins using CPEs is described in the present invention. Separation of misfolded proteins in a sample, for example to remove them from said sample, can be achieved using CPEs and has never been described before. A method to use CPEs to stain and capture a misfolded protein in a sample solution followed by sedimentation is provided. The methods using CPEs have other properties than other amyloidotrophic dyes such as congo red and ThT, most importantly CPEs provide for capture of misfolded proteins. Therefore, several methods using CPEs to capture misfolded proteins are envisioned in the present invention. The CPE can be immobilized on a solid support and thereby provide a way to capture the misfolded protein to the solid support with high selectivity and high affinity, and thereby isolate or remove it from a sample.

In a further aspect of the invention there is provided methods for capturing misfolded proteins, comprising exposing a conjugated polyelectrolyte as defined above, to a sample, whereby said conjugated polyelectrolyte selectively captures misfolded proteins in the sample.

In one aspect the present invention relates to a method for capturing the misfolded protein species in a sample on a solid support comprising the steps
- Immobilizing at least one conjugated polyelectrolyte (CPE) on a solid support
- bringing the sample in contact with the CPE
- optionally adding competition agents
- removing the solid support from the sample or separating the solid support from the sample, or
- washing the solid support without removing the captured misfolded protein from the CPE phase
- optionally detecting captured misfolded by a preferred method whereby the original sample solution then contains none or less misfolded proteins.

A further aspect the present invention relates to a method for capturing the misfolded protein species in a sample in solution comprising the steps
- bringing the sample in contact with at least one conjugated polyelectrolyte (CPE)
- optionally adding competition agents
- separation of the captured misfolded protein from the sample
- optionally detecting captured misfolded proteins by a preferred method whereby the original sample solution then contains none or less misfolded proteins.

In a further embodiment of the invention, competition agents are added to the solution to increase differentiation of CPE capturing misfolded proteins from CPE that bind, for example unspecific binding, to native or unfolded proteins. Such agents include, but are not limited to, detergents, ions, salts, chelators and solvents.

If detection of misfolded protein captured by CPE is performed, the radiation used in the method of the present invention has wavelengths in the range from about 100 nm to about 2000 nm. In one embodiment, radiation in the visible range is used. It is also possible to use multiple-photon excitation, such that instead of excitation radiation of x nm, a radiation of 2x or 3x (two-photon and three-photon excitation, respectively) is used.

In a further aspect the invention relates to a device for performing the methods according to the invention. Such a device is equipped with means for bringing the CPE, either localized on a solid support or in solution, in contact with the sample, means for optionally adding competition agents, means for optionally removing the CPE from the sample or separating the CPE from the sample, optionally washing the CPE phase without removing the captured misfolded protein from the CPE phase, optionally detecting captured misfolded by a preferred method as well as means for optionally collecting the sample solution exposed to the CPE.

Preferably the conjugated polyelectrolyte used in the present invention comprises copolymers or homopolymers of thiophene, pyrrole, aniline, furan, phenylene, vinylene, fluorene, ethylenedioxythiophene or their substituted forms, and the conjugated polyelectrolyte may have one or more ionic side chain functionalities, such as amino acids, amino acid derivatives, neurotransmittors, monosaccharides, nucleic acids, or combinations and chemically modified derivatives thereof. The ionic functionalities may comprise one or more anionic and cationic side chain functionalities.

The CPEs used in the present invention may also have end-functionalized groups where the functional group can be, but not limited to, DNA, RNA, peptides, amino acids, histidin, histidin₁₀, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads" protein coated beads, peptide coated beads, supramagnetic beads or particles, gadolinium nanoparticle, gadolinium ions, lanthanide doped nanoparticles, lanthanide-doped gadolinium oxide nanoparticles, lanthanide particles.

In a further embodiment of the present invention CPE functionalized with DNA, RNA, peptides, amino acids, histidin, histidin₁₀, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads" protein coated beads, peptide coated beads, supramagnetic beads or particles, gadolinium nanoparticle, Gadolinium ions, lanthanide doped nanoparticles, lanthanide-doped gadolinium oxide nanoparticles, lanthanide particles can be either covalent or non-covalent in nature.

In one aspect of the invention there is provided a method of detecting the separation of misassembled/aggregated forms of proteins, especially the formation of amyloid fibrils and pathogenic forms of proteins, from natural forms of proteins behind a barrier such as a tube wall, a membrane, skin, etc, comprising injecting, flowing, pumping, inhaling, transfusing or by other means exposing a sample behind a barrier, and detecting said misfolded target protein behind or inside said barrier by appropriate means of detection. Means for detection and administration of CPEs may vary depending on side-chain or end-terminal functionalization according to the present invention and are exemplified elsewhere in the description.

A further aspect of the invention provides for therapeutic methods and therapeutic agents based on conjugated polyelectrolytes for diseases related to misassembled/aggregated forms of proteins, especially the formation of amyloid fibrils and pathogenic forms of proteins, such as Alzheimer's disease, Creutzfeldt Jacob disease (CJD), variant Creutzfeldt Jacob disease (vCJD), Secondary amyloidosis, type 2 diabetes, transmissible spongiform encephalopathy (TSE, such as, CWD, Scrapie, GSS and Kuru, bovine spongiform encephalopathy (BSE).

Another aspect of the invention is a bioassay which relies on the complete distinction during the capture step of amyloid, misfolded or pathological forms of proteins in the presence of native proteins using CPE and later detected by appropriate means, such as colorimetric, absorbance or fluorescence based methods. It is very important to avoid false positives but also to avoid false negatives. Selective proteolysis of native proteins can be, but should not be required, a way to achieve this.

A still further aspect of the invention relates to the uniqueness of CPEs providing not only capture of misassembled, misfolded, aggregated or pathogenic forms of proteins, but also being capable of acting as a capture agent and at the same time report said capturing event, if desired, and be translated into optical and detectable signals.

The multiplicity of misfolded proteins that one may wish to identify also implies that the invention in a still further aspect can be implemented in the form of a microarray, and which calls for anchoring and patterning of the detecting system on a surface. The conjugated polyelectrolytes of the present invention are then used as capture agents of misfolded proteins, either in solution or on a solid support, and, if desired or required, used as a detection agent for said capture event.

The invention can also be summarised in the following aspects and embodiments:
1. A method for separation of an aggregated misfolded protein from an environment comprising a non-aggregating normal form of the protein, comprising contacting both said misfolded and normal protein with a conjugated polyelectrolyte (CPE) and separating the CPE/protein complex from the other constituents of the sample.
2. Method according to item 1, wherein the polyelectrolyte comprises copolymers or homopolymers of thiophene, pyrrole, aniline, furan, phenylene, vinylene, fluorene, ethylenedioxythiophene or their substituted forms.
3. A method according item 1 or 2, wherein said conjugated polyelectrolyte has one or more ionic side chain and/or end terminal functionalities.
4. A method according to item 3, wherein the ionic functionalities comprise one or more zwitterionic, anionic and cationic side chain functionalities.
5. A method according to item 4, wherein said ionic side chain and/or end terminal functionalities are selected from the group consisiting of amino acids, amino acid derivatives, neurotransmittors, monosaccharides, nucleic acids, DNA, RNA, peptides, amino acids, histidin, histidin 10, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads, protein coated beads, peptide coated beads, supramagnetic beads or particles, gadolinium nanoparticle, gadolinium ions, lanthanide doped nanoparticles, lanthanide-doped gadolinium oxide nanoparticles, lanthanide particles or combinations and chemically modified derivatives thereof.
6. Method according to any of the preceding items, wherein the method is performed *in vitro.*
7. Method according to any of items 1-6, wherein the method is performed *in vivo.*
8. Method according to any of the preceding items, wherein the CPE is bound to a solid support.
9. Method according to any of the preceding items, further comprising detection of the CPE/protein complex.
10. Method according to any of the preceding items, wherein binding between CPE and misfolded protein and detection of the CPE/protein complex is performed simultaneously.
11. Method according to item 9 or 10, wherein the CPE/protein complex and the detection means are on separate sides of a barrier, such as a tube wall, a membrane or skin.
12. Method for treatment of a disease caused by aggregation of misfolded proteins comprising subjecting a body fluid of a patient to a separation of an aggregated misfolded protein according to any of the preceding items.
13. Method for treatment of a disease caused by aggregation of misfolded proteins comprising administering to a patient suffering from said disease an amount of a CPE effective to inhibit further aggregation of misfolded protein or to bind intermediate forms of misfolded proteins and removing these from further reactions.
14. A pharmaceutical or diagnostic preparation comprising an optionally functionalized CPE and optionally a pharmaceutically acceptable carrier.
15. A method according to item 13, wherein said conjugated polyelectrolyte has one or more ionic side chain and/or end terminal functionalities and wherein said ionic side chain and/or end terminal functionalities are selected from the group consisting of amino acids, amino acid derivatives, neurotransmittors, monosaccharides, nucleic acids, DNA, RNA, peptides, amino acids, histidin, histidin 10, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads,, protein coated beads, peptide coated beads, supramagnetic beads or particles, gadolinium nanoparticle, gadolinium ions, lanthanide doped nanoparticles, lanthanide-doped gadolinium oxide nanoparticles, lanthanide particles or combinations and chemically modified derivatives thereof.
16. A preparation according to item 14, wherein said conjugated polyelectrolyte has one or more ionic side chain and/or end terminal functionalities and wherein said ionic side chain and/or end terminal functionalities are selected from the group consisting of amino acids, amino acid derivatives, neurotransmittors, monosaccharides, nucleic acids, DNA, RNA, peptides, amino acids, histidin, histidin 10, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads,, protein coated beads, peptide coated beads, supramagnetic beads or particles, gadolinium nanoparticle, gadolinium ions, lanthanide doped nanoparticles, lanthanide-doped gadolinium oxide nanoparticles, lanthanide particles or combinations and chemically modified derivatives thereof.

The full scope of the invention is that defined by the appended claims.

### Brief description of the drawings

Figure 1: The chemical structure of some selected examples of different CPEs based on poly-thiophene backbone and mixed poly-thiophene-benzene backbone. Side chain variations and defined backbone sizes can be constructed. These have been given a name for simplicity POWT, PTAA, POMT, PT2, tPOWT, tPTM1, f-PONT, L-POMT and D-POMT.
Figure 2: Functionalized CPEs. R1 and R2 = Functionalized at the end terminal/terminals. R1 and R2 can be the same or different. Rs = side chain.

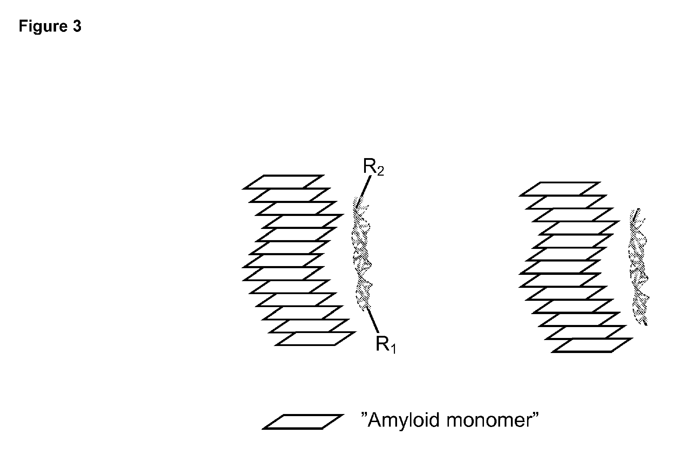

Figure 3: Schematic capture of an arbitrary amyloid polymer using either an end-terminal functionalized CPE or one without the end-terminal functionalized. The drawing is not drawn to scale and does not represent the size relationship between the CPE and the amyloid. The number of amyloid monomers in a misfolded protein can be between one and many in the present invention. Also, the CPEs can be immobilized on a solid support and examples of this are shown in later figures. The number of CPE molecules, functionalized or not, can be anything from one to many. Side chains (Rs) are not shown in the schematic.
Figure 4: Schematic drawing showing capture of misfolded protein by using a CPE immobilized on a solid support. The sample is exposed to the CPE, being functionalized or not, localized on a solid support whereby the CPE captures the misfolded proteins in said sample. The drawing is not drawn to scale and does not represent the size relationship between the CPE and the misfolded protein. The number of CPE molecules, functionalized or not, can be anything from one to many. Side chains (Rs) are not shown in the schematic.
Figure 5: Schematic drawing showing capture of misfolded protein on a suitable solid support, such as glass slide, glass bead, filter matrix, separation matrix, etc, using a CPE. The sample is exposed to the CPE, being functionalized or not, localized on a solid support whereby the CPE captures the misfolded proteins in said sample. The drawing is not drawn to scale and does not represent the size relationship between the CPE and the misfolded protein. The number of CPE molecules, functionalized or not, can be anything from one to many. Side chains (Rs) are not shown in the schematic.
Figure 6: Principle of detecting PrP-amyloid stained by the CPE PTAA using gravimetric sedimentation. The size of the crystal structure of PrP is also given as a size reference to the amount of PrP in the microscopic aggregates.

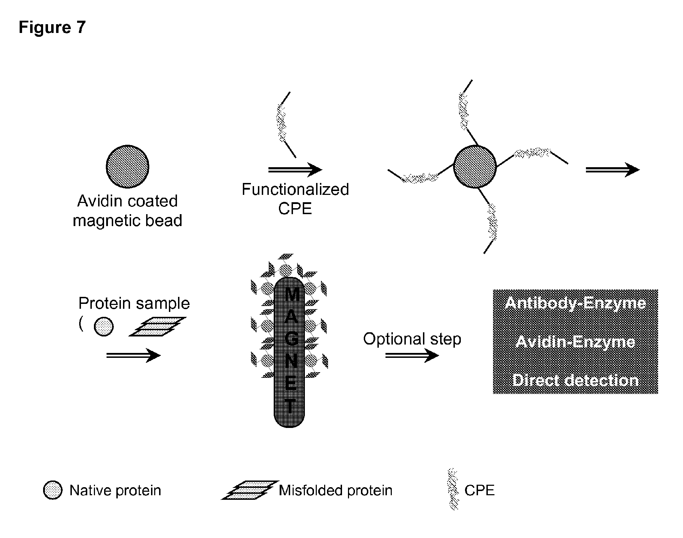

Figure 7: Schematic drawing showing capture of misfolded protein using a magnetic bead coated with a CPE. A magnetic bead, for example coated with avidin, is coated with a functionalized CPE, for example functionalized with biotin. The sample is exposed to the magnetic bead with the CPE whereby the CPE captures the misfolded proteins in said sample. The drawing is not drawn to scale and does not represent the size relationship between the CPE and the misfolded protein. A magnet can be used as a collection/concentration step. The number of CPE molecules can be anything from one to many. Side chains (Rs) is not shown in the schematic. An optional detection step can be included after the capture and the collection/concentration step using a magnet.

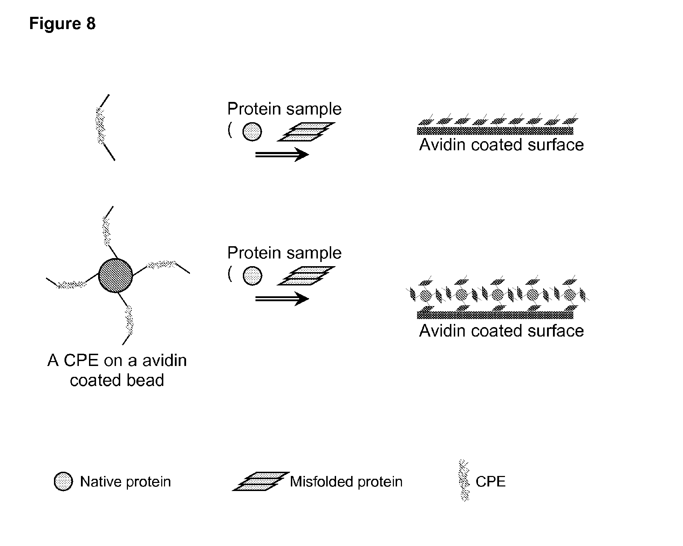

Figure 8: Schematic drawing showing capture of misfolded protein using a biotin functionalized CPE and an avidin coated surface. The functionalized CPE can be as is or on a bead. The sample is exposed to the CPE whereby the CPE captures the misfolded proteins in said sample. A avidin coated surface can be used as a collection/concentration step. The drawing is not drawn to scale and does not represent the size relationship between the CPE and the misfolded protein. The number of CPE molecules can be anything from one to many. Side chains (Rs) is not shown in the schematic. An optional detection step can be included after the capture and the surface collection/concentration step.
Figure 9. Fluorescence spectra of 0.5 mg/ml PTAA with insulin in a phosphate buffer 20 mM pH 8, before and after filtration through 0.22 µm filter.
Figure 10. Fluorescence spectra of 0.5 mg/ml PTAA with insulin containing 0, 5, 50 and 100 % fibrils in a phosphate buffer 20 mM pH 8, after filtration through 0.8 µm filter.
Figure 11. Fluorescence spectra of 0.5 mg/ml PTAA with insulin containing 0, 5, 50 and 100 % fibrils in a phosphate buffer 20 mM pH 8, after back-filtration through 0.8 µm filter.
Figure 12: Capture of fibrils using centrifugation of PTAA-fibril complexes, followed by fluorescence measurements.
Figure 13: Fluorescence spectra demonstrating the interaction between a few selected CPEs and Gd2O3 nanoparticles. The measurement was performed in double distilled water.

### Detailed description of the invention

In general terms, the present invention relates to novel methods for capturing misassembled, misfolded or aggregated forms of proteins, especially amyloid fibrils and pathogenic forms, using conjugated polyelectrolytes. The misfolded protein is exposed to the conjugated polyelectrolyte (CPE) whereby the CPE captures, binds or isolates said misfolded protein of interest. The capture can be performed with high affinity or high selectivity for the misfolded protein compared to native folded proteins. The capture is performed under conditions in which conjugated polyelectrolytes bind misfolded altered proteins, like in PrPSc or A-beta, and preferably, but not necessary, where such conjugated polyelectrolytes bind these abnormal forms but do not bind their non-aggregated normal native forms. It is desired that the non-covalent binding between the CPE and misfolded protein of interest occurs with sufficiently high affinity and under chosen conditions and sufficiently selective to be useful in assays were the aggregated, misfolded altered protein can be captured or isolated from a sample.

According to the present invention the terms capture, binding or isolation are subsets of the general term which is separation. Separation of misfolded proteins in a sample, for example to remove them from said sample, can be achieved using CPEs and has never been described before. For example, a sample solution can be flowed over or inside a solid support having a CPE/CPEs immobilized onto said solid support. The action of said CPE/CPEs is to capture the misfolded protein from the sample, but at the same time the CPE/CPEs also separates the misfolded protein from the sample.

The term "capture" as used in this application means a probe that can bind a misfolded protein, ex vivo or in vivo, and thereby separate, isolate or localize it in the presence of the non- aggregating normal form of the protein.

The invention is based on non-covalent capturing of misfolded, abnormal, misassembled, aggregating or pathogenic proteins using a conjugated polyelectrolyte interacting with said misfolded protein. The interaction occurs without covalent bonding and is based on dipole-dipole bonding, hydrogen bonding, electrostatic-and non-polar interactions between the conjugated polyelectrolyte and the misfolded protein, herein referred to as non-covalent bonding, which further includes any type of bonding that is not covalent in nature.

Some aspects of the present invention might provide for covalent attachment of conjugated polyelectrolytes to some entity, such as proteins, misfolded proteins, peptides, biomolecules, other molecules or surfaces.

One aspect of the invention is thus a process where conjugated polyelectrolytes is used for separation, for example the selective capture, binding or isolation of misfolded protein, an aggregating abnormal form or a pathological form of a protein in the presence of the native form of the protein. A sample is exposed to a conjugated polyelectrolyte, either localized on a solid support, on a bead of any size, as a hydrogel or provided as solution, whereby the conjugated polyelectrolyte captures, binds or isolates any misfolded, aggregated, misassembled or pathogenic protein. The method can be performed under such conditions that the high avidity conjugated polyelectrolyte can selectively capture said misfolded protein with high selectivity for said misfolded form in the sample containing both native and normal forms of proteins as well as the misfolded forms.

The conjugated polyelectrolyte may be immobilized on a solid support of suitable kind and applicable shape, where the surface of the solid support presents the conjugated polyelectrolyte to the sample. The shape and size of the solid support may be of any kind, for example circular, flat or a bead, and surface may be modified with conjugated polyelectrolytes by non-covalent means or covalently bonded within the structure of the solid support or attached to the surface of said solid support. A solid support according to the present invention also constitutes tubes, dialysis equipmen, pumps, insulin pumps, equipment for administrating proteins, syringes and needles. When localized on a solid support the conjugated polyelectrolyte can be in any suitable form, for example as a hydrogel, as polymer film, free standing CPEs, scaffolded or layered. The CPEs can also be provided free in solution when used as an agent to capture misfolded proteins. If the CPE is provided free in solution any means to remove the misfolded protein after being captured by the CPE can be applied, for example sedimentation, centrifugation, adsorption, absorption, drying, boiling or evaporation. Optionally the CPE can be functionalized to provide for means of detection, immobilization, facilitate capture, enhanced selectivity, enhanced affinity or other things of significance for the assay.

The described capturing, binding or isolation conditions may include the presence of a competition agent provided to the sample, which competition agent has a lesser binding avidity for the misfolded form of the protein than the conjugated polyelectrolyte. Generally, if a competition agent is used it has lesser affinity or selectivity than the conjugated polyelectrolyte.

Optionally, the surface of the solid support can be coated thereon or bonded thereto with the conjugated polyelectrolytes.

It has been reported in the scientific literature that amyloid forms of proteins, or at least protease sensitive forms of misfolded proteins, can interact with polyanions. These negatively charged polymers might be interacting with a positively charged region of the misfolded protein structure and there could be multiple interactions with the aggregated proteins. We find that a conjugated polyelectrolyte is able to bind to the misfolded protein aggregate or smaller associations of misfolded proteins, which might be even as few as two monomers of the amyloid protein, structure with much higher avidity than these polyanions, and it might therefore even displace the polyanion compounds. One possible mechanism for the high avidity of CPEs is that CPEs can form both polyionic as well as hydrophobic interactions with the misfolded protein with its charged substituents and hydrophobic conjugated backbone at the same time. Thus, conjugated polyelectrolytes immobilized to a surface or in solution can capture specifically the abnormal protein. Native proteins are non-aggregating and do not generally have such a high affinity interaction with polyanions. The assay conditions can be chosen such that the presence of lower affinity anions such as the detergent Sarkosyl improves capture specificity still further by competing with the immobilized CPE or a CPE in solution.

Separation, for example capture, binding or isolation, of a misfolded protein, an aggregating abnormal form or a pathological form of a protein in a sample using a CPE can be coupled to a change of a property of said CPE in response to the capture, binding or isolation of said misfolded protein. The changed property of the CPE can be detected by for example optical means, like fluorescence or absorption, which are then used to determine when said capturing event has occurred.

### Other methods of detection

Said conjugated polyelectrolytes can be functionalized according to aspects of the present invention to enable efficient or desired way of detecting the separation. Other methods of detecting the conjugated polyelectrolyte, that is bonded to or is in contact with said misfolded protein, is done by the appropriate method, such as two- or multiphoton spectroscopy, magnetic resonance image (MRI), PET, etc. One example to image the interaction between the CPE and the misfolded protein behind a barrier, such as a tube, a membrane, a tube, skin, etc, is described here, but there are other obvious ways. A conjugated polyelectrolyte or a functionalized conjugated polyelectrolyte can be detected by some means behind said barrier using for example two- or multiphoton spectroscopy or magnetic resonance image (MRI), is then used to determine when said separation, for example capturing or binding event, has occurred.

### CPEs as therapeutic agents

The invention also relates to the use of conjugated polyelectrolytes as novel therapeutics where one way of action is by interfering with formation of amyloid protein in vivo. The CPEs can alter amyloid pathology upon injection into organism, i.e. influence disease pathogenesis by acting as therapeutic pharmacophores. Therapeutic agents based on CPEs for protein misfolding diseases, and in particular Alzheimer's and prion diseases where an antemortem diagnosis and therapy would have a huge impact and a great benefit for public health, is accordingly envisioned in the present invention.

In the one embodiment present invention provides a method where a conjugated polyelectrolyte directly captures said misfolded protein. The capture in this case occurs without covalent bonding and is based on dipole-dipole bonding, hydrogen bonding, electrostatic-and non-polar interactions between the conjugated polyelectrolyte and the biomolecule, herein referred to as non-covalent bonding, which further includes any type of bonding that is not covalent in nature.

The term "direct capture" as used in this application means a probe that can directly capture a misfolded protein by direct binding to said misfolded protein and thereby isolate or localize it in a sample, without the need for other macromolecular compounds.

The term "avidity" as used in this application shall be taken to mean, as usual, the overall binding strength of a molecule with many binding sites with a multivalent binding agent. This should been seen in contrast to "affinity", being the binding strength between each individual binding site and of the molecule and the binding agent.

It is possible to suitable implement the conjugated polyelectrolyte, either functionalized or not, as an active part of a capture device, e. g. by immobilizing the conjugated polyelectrolyte on a substrate. The capture device comprises a suitable receptacle for said substrate, and a complex between the conjugated polyelectrolyte and the misfolded protein is formed on the substrate. Optionally, said CPE is used to detect the capturing event at the same time as it occurs.

However, other configurations are possible, e. g the conjugated polyelectrolyte can be provided in solution and passed through a flow cell while a protein solution is mixed with the flow of complex solution. The capture can be monitored by various analytical techniques.

In particular the present invention allows capturing of misfolded proteins, associated with diseases, using a conjugated polyelectrolyte interacting with the misfolded protein. Capture of misfolded proteins is essential in the development of methods for diagnosis of diseases related to misfolded proteins, to remove a misfolded protein from a sample, detecting the separation of misfolded proteins in a sample behind a barrier as well as various therapeutic techniques based on conjugated polyelectrolytes.

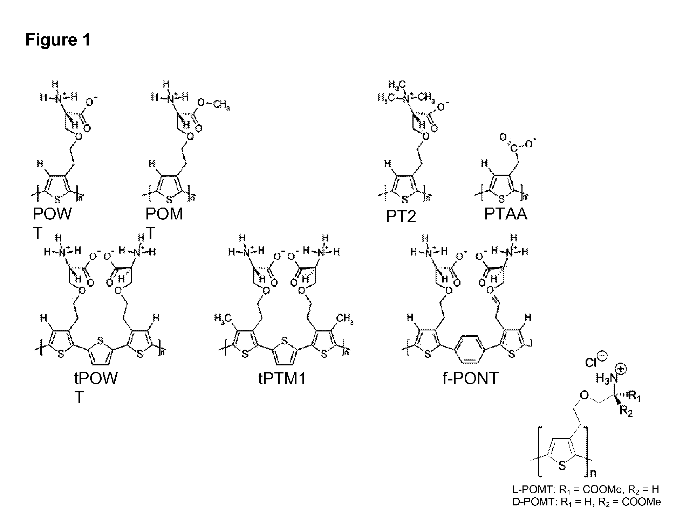

Examples of conjugated polyelectrolytes exhibiting the above discussed characteristics are poly (3- [(S)-5-amino-5-carboxyl-3-oxapentyl]-2, 5-thiophenylene hydrochloride) (POWT), polythiophene acetic acid (PTAA), poly (3-[(S)-5-amino-5-methoxycarboxyl-3-oxapentyl]-2,5-thiophenylene hydrochloride) (POMT), poly((3,3"-di[(S)-5-amino-5-carbonyl-3-oxapentyl]-[2,2';5',2"])-5,5"-terthiophenylene hydrochloride) (tPOWT), poly((1,4-di(3-[(S)-5 amino-5-carbonyl-3-oxapentyl]-thiophen-2-yl)-benzene) hydrochloride) (f-PONT), poly (3-[(R)-5-amino-5-methoxycarboxyl-3-oxapentyl]-2,5-thiophenylene hydrochloride) (D-POMT), poly (3-[(S)-5-amino-5-methoxycarboxyl-3-oxapentyl]-2,5-thiophenylene hydrochloride) (L-POMT), and other, see Figure 1. Variants, including L and D enantiomers, of these and other types of conjugated polyelectrolytes, like pyrrole, ethylenedioxythiophene, fluorene, etc, are possible in the present invention. The length of the CPE can be fixed, variable, monodisperse or polydisperse.

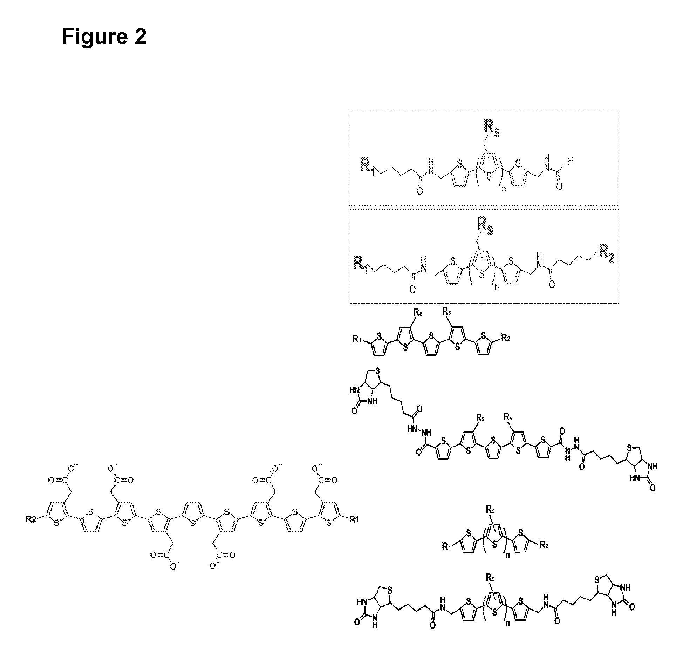

The functional groups added to the side chain (Rs) or on the end-terminals (R1 and R2), are shown in Figure 2. These can be, but are not limited to, charged anionic, cationic or zwitterionic with pKa at different pH, this makes these polythiophene derivatives suitable for forming polyelectrolyte complexes with negatively or positively charged oligomers and polymers. In addition, the ionic groups create versatile hydrogen bonding patterns with different molecules. One of the proposed reasons, the invention shall not be bound to this proposal, for CPEs unique ability for capturing misfolded proteins is the avidity. One way to look at the misfolded proteins can be seen as a polyionic molecule having an alternative or repetitive charge distribution along the axis of the molecule. The tailor made CPEs of the present invention can form strong non-covalent interactions with the misfolded proteins and therefore capture them, see the schematic illustration in figure 3. The end-terminal functionalized conjugated polyelectrolytes where the functional group can be, but is not limited to, DNA, RNA, peptides, amino acids, histidin, histidin₁₀, proteins, peptide scaffolds, biotin, avidin, streptavidin, chelators, active groups, antibodies, affinity peptide scaffolds, scFV, FAB, enzymes, ligands, receptor ligands, steroids, biomolecules or other molecules, nanoparticles, microparticles, gold nanoparticles, gold microparticles, magnetic beads, protein coated beads, peptide coated beads, supramagnetic beads or particles, gadolinium nanoparticle, gadolinium oxide particles, Gadolinium ions, lanthanide doped nanoparticles, lanthanide-doped gadolinium oxide nanoparticles, lanthanide particles, can provide for surface immobilization, secondary capture or means for detection behind a barrier according to the present invention.

The CPE may be selected to have the ability under non-selective conditions to capture both misfolded, aggregating altered or rogue forms of a protein and also the non-aggregating normal form of the protein as well as the ability to capture the misfolded protein selectively under selective conditions. The CPE, either localized on a solid support or in solution, can, if desired, under both non-selective conditions and selective conditions change its optical properties in such way that it is possible to distinguish if the CPE has captured a misfolded or native protein. The appropriate non-selective conditions or selective conditions assay conditions may be achieved by adjusting the sample, reaction or washing solution in terms of pH, ionic strength, choice of buffer or by adding salts, ions, metal ions, detergents, polyelectrolytes, proteins, particles or chelators.

It is furthermore possible to add one or more agents to the sample, reaction or washing solution capable of increasing differentiation of CPE binding the misfolded protein from CPE interacting with normal protein and in such way increase the selectivity of capturing the misfolded protein in a given sample. The CPE in this case may be provided as localized on a solid support or in solution. Such agents include detergents, such as Triton-X, Saponin, SDS, Sarkosyl, n-laurosylsarcosine, fatty acid sarcosines, CHAPS, Brij, Octyl-b-glycoside, Tween 20, Nonidet P-40 or other variants, ions and salts, such as metal ions, molecular ions and organic ions, chelators, such as EDTA, EGTA, 2,2'-Bipyridyl, Dimercaptopropanol, lonophores, Nitrilotriacetic acid, ortho-Phenanthroline, Salicylic acid and Triethanolamine, solvents, such as water, alcohols, organic solvents, chlorinated solvents, aminated solvents and sulfonated solvents, and other agents, such as polymeric materials, polyelectrolytytes (zwitterionic, anionic or cationic), Carbohydrates (including polysaccharides), Organic acids with more than one coordination group, Lipids Steroids, Amino acids and related compounds, Peptides, Phosphates, Nucleotides, Tetrapyrrols, Ferrioxamines, lonophores, such as gramicidin, monensin and valinomycin Phenolics. Other agents include proteins, such as Trypsin, Proteinase K, antibodies, serum albumine and others.

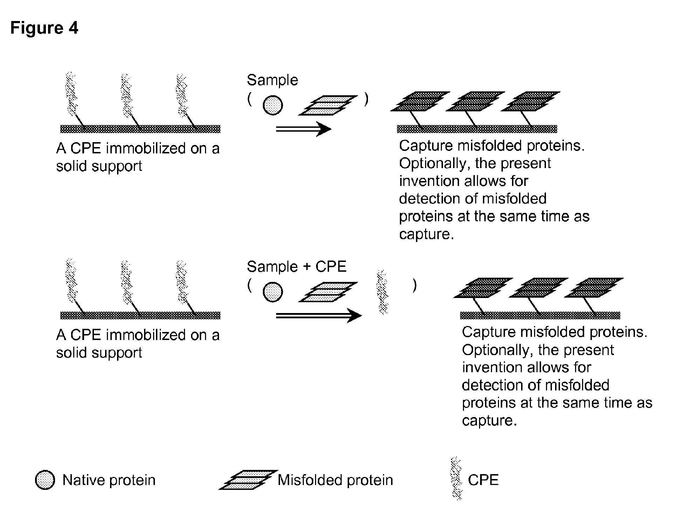

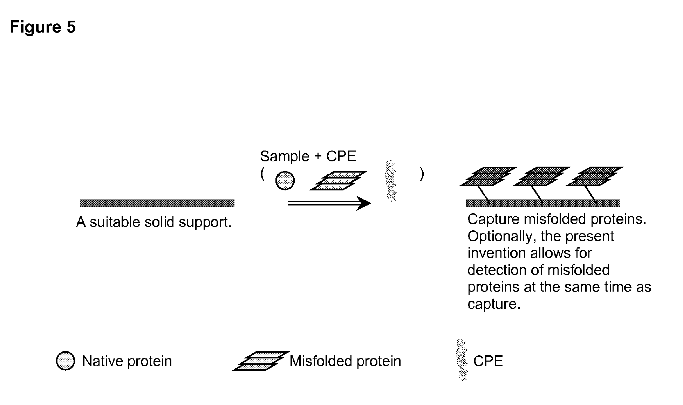

The conjugated polyelectrolyte can be suitably implemented as an active part of a device that capture, isolate, bind or remove misfolded proteins in a sample. This can be achieved by, for example, immobilizing the conjugated polyelectrolyte on a substrate which then is exposed to a sample by appropriate means (see Figure 4 and 5 for two examples). Suitably the device comprises a suitable receptacle for said substrate, and an interaction between the conjugated polyelectrolyte and the misfolded protein is formed on the substrate whereby it captures, isolates, binds or removes misfolded proteins in the sample. The conjugated polyelectrolyte can be a part of a system using an antibody that recognizes or identifies the misfolded protein (for example PrP^{SC}, PrPres, amyloid-beta, fibrils formed of Aβ(1-40), Aβ(1-42) or Aβ(1-43), amyloid-β (Aβ) peptides, beta-2-microglobulin molecules in dialysis-related amyloidosis (DRA), IAPP, alpha-synuclein in Parkinson's disease or huntingtin in Huntington's disease) that has been or will be captured by the CPE. Optionally, the conjugated polyelectrolyte can be used as an optical probe to report the capture of the misfolded protein (see Figure 4 and 5 for two examples). However, other configurations are possible, e.g. the conjugated polyelectrolyte can be provided in solution for capturing, and at the same time staining, a misfolded protein in a sample or in vivo. An antibody, in solution or on a solid support, can then be used identify or recognize the misfolded protein.

The detailed description of the invention that follows will deal separately with the conjugated polyelectrolytes, misfolded, abnormal, aggregating, rouge or pathogenic protein, diseases related to said misfolded proteins, methods of capturing said misfolded proteins from a sample solution, immobilization of conjugated polyelectrolytes onto solid supports, and arrays. The invention is finally exemplified with a number of experiments demonstrating the utility thereof.

### I Conjugated polyelectrolytes

The present invention relates to a variety of conjugated polyelectrolytes, with a minimum of 3 mers, consisting of mers derived from the monomers thiophene, pyrrole, aniline, furan, phenylene, vinylene, fluorene, ethylenedioxythiophene or their substituted forms, forming homopolymers and copolymers thereof. The conjugated polyelectrolyte can be mono dispersed, consist of polyelectrolyte chains with a well-define chain length, or poly dispersed, comprise of polyelectrolyte chains with different chain length. Furthermore, monomers with anionic-, cationic or zwitterionic side chain functionalities are included within the scope of the invention. The side chain functionalities is derived from, but not limited to, amino acids, amino acid derivatives, neurotransmittors, monosaccharides, nucleic acids, or combinations and chemically modified, L and D enantiomers, or derivatives thereof. The conjugated polyelectrolytes of the present invention may contain a single side chain functionality or may comprise two or more different side chain functionalities. The functional groups of the conjugated polyelectrolytes, charged anionic or cationic at different pHs, make these polyelectrolyte derivatives suitable for forming strong polyelectrolyte complexes with negatively or positively charged oligomers and polymers. In addition, the ionic groups create versatile hydrogen bonding patterns with different molecules.

Some aspects of the present invention might provide for covalent attachment of conjugated polyelectrolytes to some entity, such as proteins, misfolded proteins, peptides, biomolecules or other molecules.

The functional groups added to the side chain (Rs) or on the end-terminals (R1 and R2), in Figure 1 or Figure 2, can be, but not limited to, charged anionic, cationic or zwitterionic with pKa at different pH, this make these polythiophene derivatives suitable for forming polyelectrolyte complexes with negatively or positively charged oligomers and polymers. In addition, the ionic groups create versatile hydrogen bonding patterns with different molecules. One of the proposed reasons, the invention shall not be bound to this proposal, for CPEs unique ability for capturing misfolded proteins is the avidity. One way to look at the misfolded proteins can be seen as a polyionic molecule having an alternative or repetive charge distribution along the axis of the molecule. The tailor made CPEs of the present invention can form strong non-covalent interactions with the misfolded proteins and therefore capture them, see the schematic in figure 3. The end-terminal functionalized conjugated polyelectrolytes where the functional group can be, but not limited to, DNA, RNA, peptides, amino acids, histidin, histidin₁₀, proteins, peptide scaffolds, biotin, avidin, streptavidin, chelators, active groups, antibodies, affinity peptide scaffolds, scFV, FAB, enzymes, ligands, receptor ligands, steroids, biomolecules or other molecules, nanoparticles, microparticles, gold nanoparticles, gold microparticles, magnetic beads, protein coated beads, peptide coated beads, supramagnetic beads or particles, gadolinium nanoparticle, gadolinium oxide particles, Gadolinium ions, lanthanide doped nanoparticles, lanthanide-doped gadolinium oxide nanoparticles, lanthanide particles, can provide for surface immobilization, secondary capture or means for detection behind a barrier according to the present invention.

### II Misfolded protein

The conjugated polyelectrolytes of the present invention capture a misfolded protein of interest. In the present invention the term misfolded protein should be taken it its broadest sense, including misassembled proteins, pathological or rogue forms of proteins, i.e. amyloid form, misfolded form or aggregated forms of protein, as plaques, preferably the misfolded proteins can be amyloids which are insoluble fibrous protein aggregations sharing specific structural traits.

The capture occurs without covalent bonding and is based on dipole-dipole bonding, hydrogen bonding, electrostatic-and non-polar interactions between the conjugated polyelectrolytes and the misfolded protein. The conjugated polyelectrolyte might interact with both a normal native protein and the misfolded protein. The conjugated polyelectrolytes can also capture amyloid or misfolded proteins prepared in vitro. Without being bound by theory, it is the present hypothesis that the conjugated polyelectrolyte can capture misfolded specifically and with high affinity under suitable assay conditions. The misfolded protein can be chemically modified to be captured by the conjugated polyelectrolyte of choice. Methods of derivatizing a diverse range of proteins are well known. For example, amino acid side chains can easily be modified to contain polar and non-polar groups or groups with hydrogen bonding abilities. The misfolded protein can be in solution, on a solid phase, in vivo, in vitro or in tissue samples and the capture of misfolded proteins can be made in water solutions, organic solvents, body fluids, on a solid phase or in tissue samples.

Samples, either *in vivo* or *in vitro*, of interest, that may contain misfolded proteins, include, but is not limited to, blood, brain, peripheral tissue, homogenized brain, homogenized peripheral tissue, fluids, biopsies, urine, cerebrospinal fluid (CSF), lymph and other samples of relevance. Assays based on capture of misfolded proteins are of great commercial interest, of huge diagnostic value, as therapeutic agents as well as selective assays for capture of misfolded proteins in blood (screening of blood products) or other complex media. Staining of tissue sections, from brain, muscle, fat, mucus, nerve, blood vessels or other tissues.

### III Diseases related to misfolded proteins

Diseases featuring amyloid proteins are relevant examples for the description of diseases related with misfolded proteins, where amyloidosis are known as a disease and may be inherited or acquired. Note that amyloidosis by default usually refers to AA amyloidosis, but any disease related to amyloid proteins, which presents amyloid deposition, is an amyloidosis. For example CJD, vCJD, Alzheimer's and diabetes are almost never referred to as amyloidoses.

In this paragraph some examples of amyloidosis with relevance to the present is named. Primary amyloidosis includes mutations in lysozyme, transthyretin, apolipoprotein B, fibrinogen and AL amyloidosis (immunoglobulin light chains, as seen with multiple myeloma). Secondary amyloidosis includes AA amyloidosis (serum amyloid A protein, an acute-phase protein due to chronic inflammation) and Gelsolin amyloidosis (plasma gelsolin fragments). Familial or hereditary amyloidosis, are most commonly caused by mutations in the transthyretin protein, but in rare occurrences can also be caused by apolipoprotein A1, gelsolin, fibrinogen, and lysozyme mutations, primarily caused by genetics, believed to be autosomal dominant, high probability of passage to offspring, Appalachian type amyloidosis and Shar Pei fever for amyloidosis in Shar Peis. Examples of organ-specific amyloidosis are Diabetes mellitus type 2 (amylin, also known as IAPP), Neurology, Alzheimer's disease (Aβ 39-42), Parkinson's disease (alpha-synuclein), Huntington's disease (huntingtin), Transmissible spongiform encephalopathies (prion protein, PrP), some examples are Creutzfeldt-Jakob disease (PrP in cerebrum), Kuru (diffuse PrP deposits in brain), Fatal Familial Insomnia (PrP in thalamus) and Bovine spongiform encephalopathy (PrP in cerebrum of cows), Congophilic angiopathy (Amyloid beta). Cardiac amyloidosis includes congestive heart failure; some instances (PrP or transthyretin in heart). Inclusion body myositis. Another important example are the Iatrogenic conditions like insulin amyloidosis, believed to be caused by injection-administered insulin.

Some non-disease amyloids are native amyloids in organisms, Curli E. coli Protein (curlin), Yeast Prions [Sup35], Podospora Anserina Prion Het-s, Malarial coat protein, spider silk, Mammalian melanosomes (pMel), Tissue-type plasminogen activator (tPA), a hemodynamic factor, Calcitonin and proteins and peptides engineered to make amyloid.

The prion diseases [e.g. bovine spongiform encephalopathy (BSE), and Creutzfeldt-Jakob disease (CJD)], are associated with the conformational conversion of the normal cellular prion protein, (PrP^{C}), to an infectious disease-associated isoform denoted PrP^{Sc}. The misfolded infectious form of the protein, PrP^{sc} is the cause of a group of rare, fatal brain diseases, called prion diseases that affect humans and mammals. The prion diseases are also known as transmissible spongiform encephalopathies (TSE), and they include bovine spongiform encephalopathy (BSE, or "mad cow" disease) in cattle; scrapie in sheep; chronic wasting disease in deer and elk; and in humans [Creutzfeldt Jakob disease (CJD), Gerstmann-Sträussler-Scheinker disease (GSS), Kuru]. The conjugated polyelectrolytes of the present invention are intended to be used for methods for capture of pathogenic prions associated with these diseases.

### IV Methods of detection

As already indicated the present invention is based on the utilization of conjugated polyelectrolytes, functionalized conjugated polyelectrolytes or combinations thereof to capture, bind, isolate or remove a misfolded protein from a sample. The capture event can be observed for example by, but not limited to fluorescence, Förster resonance energy transfer (FRET), quenching of emitted light, absorption, two- or multiphoton spectroscopy, magnetic resonance image (MRI), dynamic light scattering (DLS), an electrical or magnetic response, electrochemistry, amperometry, coulometry, gravimetry, polarization or anisotropy, fluorescence correlation spectroscopy (FCS), quartz crystal microbalance, quartz crystal microbalance with dissipation or other techniques and physical properties. The emission intensities can be recorded by a fluorometer and enhancement of the photon flow in the detector can increase the sensitivity. This can be achieved using a lamp or a laser as the excitation source. The fluorometric change can also be detected by the use of a fluorescence microscope or a confocal microscope. A combination of excitation or emission filter can be used and the picture can be recorded by a CCD-camera, video camera, regular camera or by a Polaroid camera. The pictures can then be analyzed by image processing software on a computer, Image correlation spectroscopy (ICS) or by other means.

### V Immobilization of conjugated polyelectrolytes and proteins

The conjugated polyelectrolytes, biomolecules, misfolded proteins, antibodies, proteins, peptides, misfolded proteins or other molecules used in the present invention can be immobilized on a variety of solid supports, including, but not limited to silicon wafers, glass (e. g. glass slides, glass beads), glassy carbon, silicon rubber, polystyrene, polyethylene, polypropylene, teflon, silica gel beads, gold, indium tin oxide, filter paper (e. g. nylon, cellulose and nitrocellulose), standard copy paper or variants and separation media, other chromatographic media, magnetic beads or particles, supramagnetic beads or particles, gadolinium, gadolinium oxide particles or other solid supports useful in the present invention. Transfer of the conjugated polyelectrolyte to the solid support can be achieved by using i. a. but not limited to, dip coating, spin-coating, contact printing, screen printing, ink jet technologies, spraying, dispensing and microfluidic printing by the use of soft lithography or the BIACORE™ (Biacore, Uppsala, Sweden) system.

Immobilization of the conjugated polyelectrolytes is achieved by physical adhesion or covalent attachment to the solid support, and can be performed at elevated temperatures or by entrapment in a hydrogel matrix. Immobilization of the conjugated polyelectrolytes of the present invention may be desired to improve their ease of use, assembly into devices (e. g. arrays), stability, robustness, fluorescent response, to fit into the process of high-throughput-screening (HTS) using micro titer plates and other desired formats. Solvents for the conjugated polyelectrolytes of the present invention and the prion proteins during the immobilization to the solid support can be, but are not limited to, water, buffered water solutions, methanol, ethanol and combinations thereof. Supporting polymers of other kinds can also be added in this step. Biomolecules, misfolded proteins, antibodies, proteins, peptides, misfolded proteins or other molecules can also be immobilized on a solid support or in microtiter wells. A recognizing, identifying or capture antibody, or other biomolecules with selective capabilities, can also be immobilized, either by its own, together with the conjugated polyelectrolyte (i. e. mixed with the polyelectrolyte solution) or later recognizing the CPE that has captured a misfolded protein. When the biomolecules, misfolded proteins, antibodies, proteins or peptides are immobilized on the solid support together with the conjugated polyelectrolyte of the present invention they form a complex with the polyelectrolyte through non-covalent interactions. This complex is formed without covalent chemistry and is based on hydrogen bonding, electrostatic-and non-polar interactions between the conjugated polyelectrolyte and the prion protein.

### VI Arrays and lines

According to the present invention the generation of large arrays of the same or different conjugated polyelectrolytes in each spot or line can overcome shortcomings of a single sensor or a solution based approach. The array or parallel line approach opens up the parallel analysis of one or different samples that may contain misfolded proteins to one or different-conjugated polyelectrolytes in an easy way, where at least one step in the protocol utilizes a CPE to capture the misfolded protein. The main purpose of using arrays is to increase ease of use, portability, quantification, selectivity among other qualities and characteristics. With this approach we can explore the ability to measure multicomponent samples and to use partially selective sensor spots. This gives the opportunity to analyze two or more samples of interest at the same time and to do on-chip determinations. By immobilizing the conjugated polyelectrolyte and/or biomolecules, misfolded proteins, prion protein, antibodies, proteins or peptides on solid supports of any size and in any chosen patterns (such as arrays, lines, spots, posts) small, portable, easily read and interpretable devices can be constructed.

The use of multiple arrays requires that detection can be done for a great number of samples, more or less simultaneously. This is often done in the form of a microarray, where many individual detector elements (or probes) are integrated on a small surface area, to allow for massive parallelism in the detection. We have shown that the conjugated polyelectrolyte and the conjugated polyelectrolyte/protein complexes can be printed by micro contact printing using elastomer stamps. Transfer onto a microarray surface may also be done by spotting conjugated polyelectrolyte solutions, or by ink jetting polyelectrolyte solutions or by the other methods mentioned above. These steps are essential to prepare a multipixel microarray.

### Examples

### Example 1. Capture and detection of PrP-amyloid in solution using conjugated polyelctrolytes. Surface detection by means of fluorescence microscopy

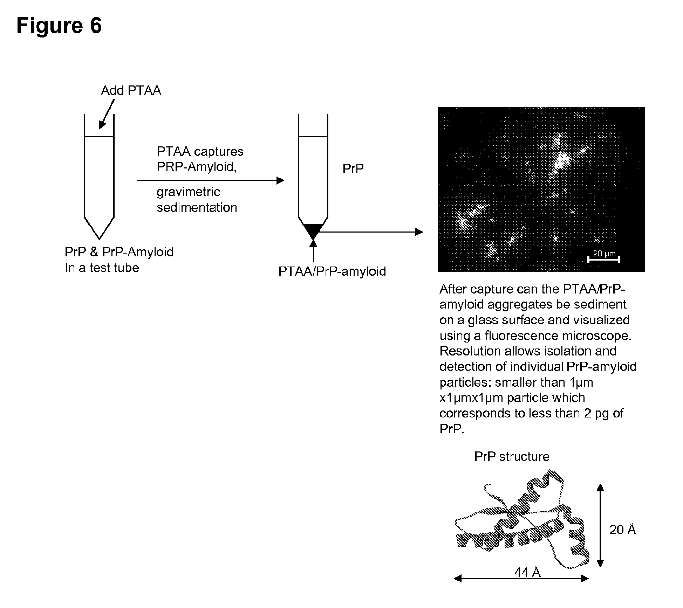

PrP and PrP-amyloid sample, the latter being PrP^{Sc}- or PrP^{res}-like, is contained in a test tube. PrP-amyloid was generated through dialysis of 1 mg/ml recHuPrP90-231 dissolved in 3 M guanidinium hydrochloride versus water. Another protocol to generate PrP-amyloid is by shaking in a test tube at a high salt concentration. Adding the conjugated polyelectrolyte PTAA (10 µg/ml) to this solution, containing PrP and PrP-amyloid, causes PTAA to capture, bind and stain as well, PrP-amyloid. The PTAA/PrP-amyloid formation can then be left to sediment to the bottom of the test tube, and can then easily be isolated from the sample and detected if desired. Other ways to collect PTAA/PrP-amyloid is, but not limited to, filtration, centrifugation, capture on a hydrophobic surface, capture on a surface with covalently attached PTAA, using functionalized particles, salt precipitiation and by immunoprecipitation. Methods for detection or visualization of PTAA/PrP-amyloid includes, but not limited to, fluorescence microscopy, fluorescence detection in plate readers or spectrofluorometers, absorption detection in plate readers or spectrometers, array fluorescence reader, photodiodes, fluorescence polarization or anisotropy, circular dichroism and more. This method also provides a way to capture, clean, remove or filter PrP-amyloid, PrP^{Sc}, PrP^{res} from a given sample in solution. In this particular example PTAA/PrP-amyloid formation was collected on a glass substrate and visualized using a fluorescence microscope, see figure 6. Using this instrument the resolution allows identification of individual PrP-amyloid particles smaller than 1 µm x 1 µm x 1 µm which corresponds to less than 2 pg of PrP.

### Example 2: filtration, capture

In order to separate native and fibril insulin and hence purifying the fibril insulin, filtration of polymer-insulin samples were performed. Samples tested were native insulin and fibril insulin, with and without PTAA. Initial PTAA concentration were 0.5 mg/ml and insulin concentration 2 mg/ml, and samples contained 10 µl PTAA + 25 µl insulin to 1 ml in phosphate buffer 20 mM pH 8. For all samples fluorescence measurements were performed both before and after filtration, and the filters were opened and visually inspected. Filters used were 0.22 µm Millex-gu, and 1 ml of every sample were filtered through the filters.

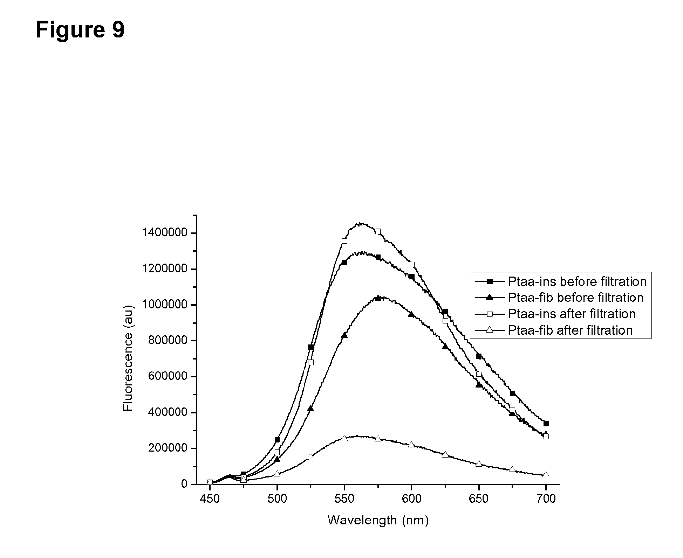

The fluorescence spectra of PTAA - native insulin (PTAA-ins) and PTAA - fibril insulin (PTAA-fib) are shown in figure 9 before and after filtration through the 0.22 µm filter. Before filtration the spectra look as expected. After filtration it is obvious that samples with PTAA and fibril insulin is retained in the filter (seen as a decrease in fluorescence), while samples with native insulin does not affect the passing of the polymer. Thus, filtration could be used to remove native insulin bound to PTAA. A visual inspection of the filters after filtration show that aggregates of PTAA - fibril insulin is present on the filters (seen as red aggregates), while filters with PTAA - native insulin show no aggregates or change in colour.

Measurements to detect the presence of PTAA - fibril insulin complexes caught in the filter were also done. Samples with PTAA alone and with native insulin containing 0, 5, 50 and 100 % fibril insulin were prepared as described above. The content of native insulin were decreases as the content of fibril insulin increased, resulting in the same total insulin content in all samples. The samples were filtrated through a 0.8 µm Millex-aa filter and fluorescence was measured on the filtrate. The filters were then back-filtered, by pressing 1 ml buffer through the filters backwards. Fluorescence was then measured on the back-filtered filtrate.

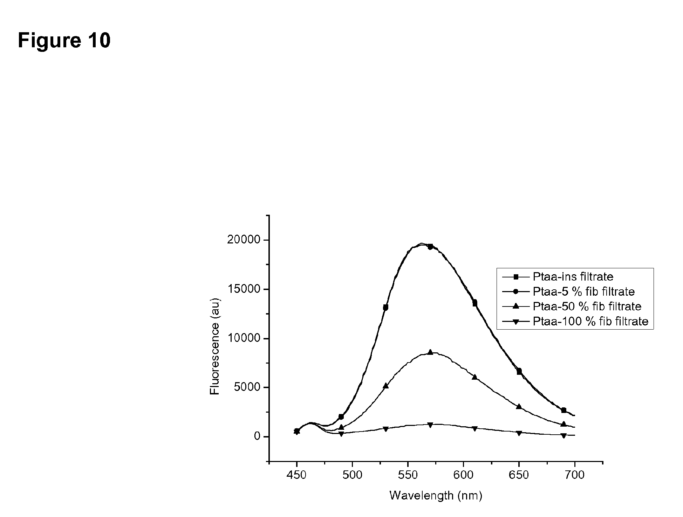

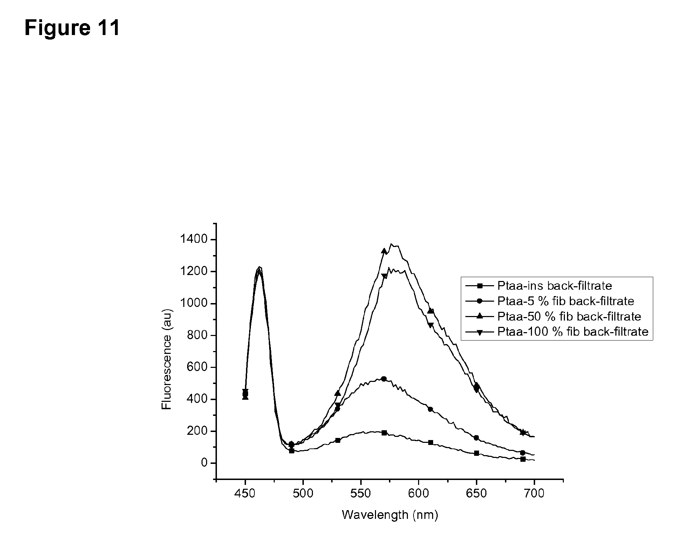

The fluorescence spectra of the first filtrate is shown in figure 10. Increasing fibril content leads to more PTAA - fibril insulin complexes being retained in the filter, while PTAA can pass freely with native insulin. The back-filtered samples are shown in figure 11. While most complexes are still caught in the filters, it is obvious that increasing fibril content leads to more PTAA - fibril insulin complexes being resolved into solution following back-filtration. Thus PTAA can be used to capture fibril insulin.

Using filtration to capture PTAA - fibril complexes is not limited to the filters and examples above. Any other type of filter, matrix, buffers, concentrations, and additives can be used, and analysis and detection can be obtained by fluorescence measurements, absorbance measurements, visual inspection, microscopic methods, fluorescence polarization or anisotropy, circular dichroism and more.

### Example 3:

### Capture and detection of Insulin-amyloid in solution using conjugated polyelectrolytes. Solution detection by means of fluorescence measurements.

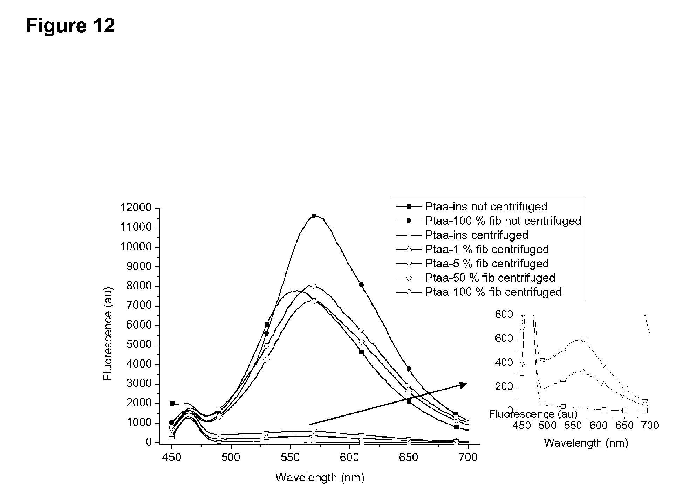

Samples with native insulin containing 0, 1, 5, 50 and 100 % fibrils (2 mg/ml, 25 µl) are mixed with PTAA (0.05 mg/ml, 10 µl) and 965 µl 20 mM phosphate buffer pH 7.0 in a test tube. Fibril insulin was generated through incubation of native insulin at 65 degrees Celsius at pH 2 for 8 hours. The samples were centrifuged at 10 000 rpm for 5 minutes, the supernatant was removed, new buffer was added, and the procedure was repeated once. The PTAA binds to the fibril insulin and sediments to the bottom of the test tube under the centrifugation. The samples were then measured using a fluorescence microplate reader. Reference samples of PTAA - native insulin and PTAA - fibril insulin that had not been centrifuged were also measured. The results are shown in figure 12.

The PTAA-fibril aggregates sediments to the bottom of the test tube and can easily be collected for detection by removing the supernatant following centrifugation. By resolving the pellet the PTAA-fibril complexes can then be detected by fluorescence measurements. Methods for detection or visualization of PTAA/PrP-amyloid includes, but not limited to, fluorescence microscopy, fluorescence detection in plate readers or spectrofluorometers, absorption detection in plate readers or spectrometers, array fluorescence reader, photodiodes, fluorescence polarization or anisotropy, circular dichroism and more. This method also provides a way to capture, clean, remove or filter amyloids and fibrils from a given sample in solution. As can be seen in this example, increasing the amount of fibrils leads to more PTAA-fibril complexes. From 1 % fibrils up to 100 % fibrils the fluorescence intensity increases, showing the binding between PTAA and the fibrils, as no PTAA at all is present following centrifugation with native insulin.

### Example 4: Immobilization of CPEs to solid supports

This example describes the immobilization of PTAA to a glass surface that has been coated with APTES. The silane APTES was in order to have amine groups for the chemical coupling. EDC (or EDAC) is a zero-length crosslinking agent used to couple carboxyl groups to primary amines. This crosslinker can be used in diverse applications such as forming amide bonds in peptide synthesis, attaching haptens to carrier proteins to form immunogens, labeling nucleic acids through 5' phosphate groups and creating amine-reactive NHS-esters of biomolecules.

The CPEs having a carboxyl group, -COOH, (such as PTAA, POWT, tPTAA or tPOWT) can be activated first and then coupled to an amine surface of any kind, such as functionalized magnetic beads. CPEs that does not contain any carboxyl groups but have amine groups (such as POMT, tPOMT and PTT) can be coupled to an activated carboxyl surface. If you have a magnetic bead with carboxyl groups just put this one into the EDC/NHS solution instead.

### Protocol, immobilization of PTAA

- Clean a glass slide using TL1 wash (5 parts H₂O, 1 part 25 % NH₃, 1 part 28 % H₂O₂, heat to 85° Celsius for 5 minutes, rinse with water) and TL2 wash (6 parts H₂O, 1 part 37 % HCl, 1 part 28 % H₂O₂, heat to 85° Celsius for 5 minutes, rinse with water) to remove organic and inorganic substances and to make them hydrophilic. Dry with N₂.
- Put glass slide in a vaporization chamber. Place 200 µl APTES in the chamber, vaporize at 60° Celsius for 10 minutes, bake at 150° Celsius for 60 minutes. Rinse in xylene and store in xylene. The amount of APTES might be different for different vaporization chambers.
- Prepare a mixture of 2 mM EDC and 1 mM NHS in H₂O.
- Mix 1 part EDC/NHS with 1 part PTAA.
- Apply 100 µl PTAA-EDC/NHS on the glass slide, incubate covered in darkness for 30 minutes.
- Wash surface 3 times with 20 mM phosphate buffer pH 8. Dry with N₂.

PTAA will be covalently bound to the surface in a thin layer, and can be used to bind molecules of interest. Some modifications to the protocol might be used, like concentrations, pH, buffers, incubation times and materials used. The concentrations of EDC/NHS have been selected to activate only a few of the carboxylic groups on the PTAA, to increase the amount of PTAA bound to the surface and to preserve the binding properties of the PTAA. Preferably only one side-group of the polymer should be activated. It has not been evaluated if the reported concentrations are optimal. The pH and the buffer composition used for washing can be modified. In the protocol for Covalink microtiter plates by Nunc the washing buffer is 116.9 g NaCl, 10 g MgSO₄*7H₂O, 0.5 ml Tween 20, 1 litre PBS. The incubation times can be modified to improve the results. Sulfo-NHS can be used instead of NHS as it is somewhat more stable.

### Protocol, immobilization of POMT

As POMT does not contain a carboxylic acid side-group, the procedure for immobilization is reversed to that of PTAA. Instead of a APTES surface a surface containing Carboxyl groups should be used, such as a PEG matrix. The PEG matrix is treated with BrCH₂COOH and NaOH to introduce COOH groups, after which EDC/NHS is applied to the surface to activate the carboxylic groups before POMT is added. Other surfaces could also be used. The procedure has not been evaluated. See paper "Photografted Poly(ethylene glycol) Matrix for Affinity Interaction Studies", Biomacromolecules, Vol 8, No.1, p. 287-295, 2007 for method description.

### Protocol, immobilization of POWT and PTT

As POWT and PTT contain both a carboxylic and an amine group, using EDC/NHS can cause crosslinking between polymer chains instead of between the polymer and the surface, which is not desired. However, by tuning the concentration of EDC/NHS and polymer, it might be possible to create a thicker, crosslinked polymer layer covalently attached to the surface and even better results. Either an APTES surface or a surface containing Carboxyl groups could be used. The procedure has not been evaluated.

### Surfaces

In the examples above a glass slide has been used for immobilization. Other surface can also be used, such as silicon wafers, glass beads, microtiter plates

(Nunc's Covalink are pre-coated with an APTES surface), or any other surface with the right properties.

### Other immobilization techniques

Other immobilization techniques can also be used. For covalent attachment, functionalized polymers with biotin or other terminal groups can be used. For non-covalent attachment, physisorbtion to a protein layer on a surface can be used. Polymer can for example be coated on Nunc Maxisorp microtiter plates coated with dextran sulphate. Any other techniques that will yield a polymer coated surface can also be used.

### Adding samples to the surfaces

The sample to be evaluated or captured can be added to the polymer coated surface in different manner. A volume of sample can simply be added to the surface, incubated for an appropriate time and rinsed. Flow canals can be used to lead the sample on to the surface in the proper amount, which will yield a more repeatable system. If beads are used, the can be added to the sample solution, incubate and be separated from the solution using centrifugation, magnetic separation, or any other separation technique.

### Example 5: Lanthanide nanoparticles coated with conjugated polyelectrolytes

This example shows that a specific interaction between CPEs and lanthanide nanoparticles, such as Eu203, Gd2O3 or Nd2O3, is possible. These particles, which can be anything from a few nanometer in size to a few hundreds of nanometer, might in turn be useful for in vivo imaging of misfolded proteins and diseases related to misfolded proteins according to the present invention. Modification of the lanthanide particles is possible, such as doping with various metals, such as Fe3+, Eu3+, Tb3+, etc. Before coating or binding CPEs it is possible to pre-coat the lanthanide particles by for example biomolecules, diethylene glycol, alkanes, carboxyl groups, citric acid, amine groups etc before further adding other molecules.

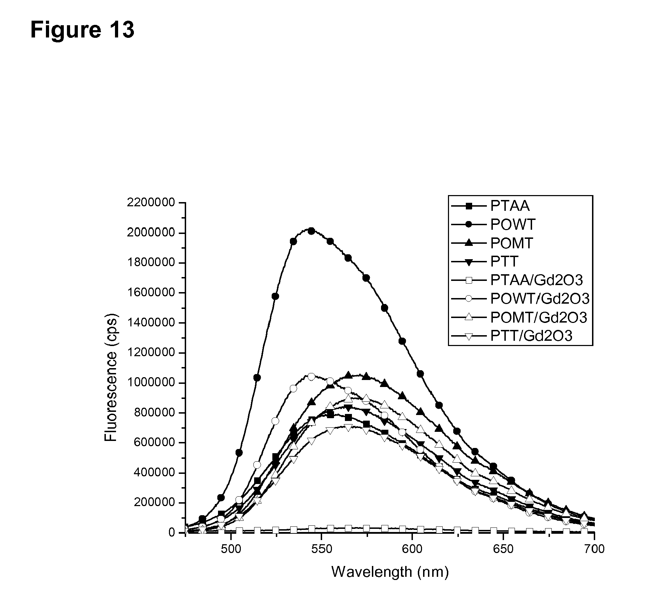

To demonstrate the interaction between a few selected CPEs and Gd2O3 nanoparticles the fluorescence of the CPEs was recorded, see figure 13. Excitation wavelength was set to 400 nm and the CPE measurement concentration was 2. 5 µg/mL and the gadolinium oxide particles was diluted 200 times from stock solution, bought from Sigma-Aldrich. The measurement presented here was performed in double distilled water but can be performed in many kinds of buffer solutions or in a complex media.

## Claims

1. A method for separation of an aggregated misfolded protein from an environment comprising a non-aggregating normal form of the protein, comprising contacting both said misfolded and normal protein with a conjugated polyelectrolyte (CPE) and separating the CPE/protein complex from the other constituents of the sample.

2. Method according to claim 1, wherein the polyelectrolyte comprises copolymers or homopolymers of thiophene, pyrrole, aniline, furan, phenylene, vinylene, fluorene, ethylenedioxythiophene or their substituted forms.

3. A method according claim 1 or 2, wherein said conjugated polyelectrolyte has one or more ionic side chain and/or end terminal functionalities, such as one or more zwitterionic, anionic and cationic side chain functionalities.

4. A method according to claim 3, wherein said ionic side chain and/or end terminal functionalities are selected from the group consisiting of amino acids, amino acid derivatives, neurotransmittors, monosaccharides, nucleic acids, DNA, RNA, peptides, amino acids, histidin, histidin 10, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads, protein coated beads, peptide coated beads, supramagnetic beads or particles, gadolinium nanoparticle, gadolinium ions, lanthanide doped nanoparticles, lanthanide-doped gadolinium oxide nanoparticles, lanthanide particles or combinations and chemically modified derivatives thereof.

5. Method according to any of the preceding claims, wherein the method is performed *in vitro*.

6. Method according to any of claims 1-4, wherein the method is performed *in vivo*.

7. Method according to any of the preceding claims, wherein the CPE is bound to a solid support.

8. Method according to any of the preceding claims, further comprising detection of the CPE/protein complex.

9. Method according to any of the preceding claims, wherein binding between CPE and misfolded protein and detection of the CPE/protein complex is performed simultaneously.

10. Method according to claim 8 or 9, wherein the CPE/protein complex and the detection means are on separate sides of a barrier, such as a tube wall, a membrane or skin.

11. A diagnostic preparation comprising an optionally functionalized CPE and optionally a pharmaceutically acceptable carrier.

12. A preparation according to claim 11, wherein said conjugated polyelectrolyte has one or more ionic side chain and/or end terminal functionalities and wherein said ionic side chain and/or end terminal functionalities are selected from the group consisting of amino acids, amino acid derivatives, neurotransmittors, monosaccharides, nucleic acids, DNA, RNA, peptides, amino acids, histidin, histidin 10, proteins, peptide scaffolds, biotin, avidin, streptavidin, chelators, active groups, antibodies, enzymes, ligands, receptor ligands, steroids, biomolecules or other molecules, nanoparticles, microparticles, gold nanoparticles, gold microparticles, magnetic beads,, protein coated beads, peptide coated beads, supramagnetic beads or particles, gadolinium nanoparticle, gadolinium ions, lanthanide doped nanoparticles, lanthanide-doped gadolinium oxide nanoparticles, lanthanide particles or combinations and chemically modified derivatives thereof.
